# EUROPEAN PATENT SPECIFICATION

(11) **EP 4 667 494 B1**
(45) Date of publication and mention of the grant of the patent: **09.12.2026**
(21) Application number: 25212478.9
(22) Date of filing: 26.10.2023
(51) Int. Cl.: C07K 16/28

(54) **CAR T-CELLS AGAINST CD79B FOR THE TREATMENT OF NON-HODGKIN LYMPHOMA**
CAR-T-ZELLEN GEGEN CD79B ZUR BEHANDLUNG VON NON-HODGKIN-LYMPHOM
LYMPHOCYTES T CAR DIRIGÉS CONTRE CD79B POUR LE TRAITEMENT DU LYMPHOME NON HODGKINIEN

(43) Date of publication of application: 24.12.2025
(62) Document number(s) of the earlier application(s) in accordance with Art. 76 EPC: 23383097.5 / 4 545 085
(73) Proprietor: Fundación Instituto de Investigación Sanitaria Fundación Jiménez Díaz, 28015 Madrid (ES)
(72) Inventor: MARTÍN ANTONIO, Araceli Beatriz, 28040 Madrid (ES); LLAMAS, Pilar, 28040 Madrid (ES); SERRANO LÓPEZ, Juana, 28040 Madrid (ES); SOLÁN BLANCO, Laura, 28040 Madrid (ES)
(74) Representative: Hoffmann Eitle

(56) References cited:
- WO-A1-2019/241688
- WO-A1-2021/222944
- US-A1- 2021 145 878
- ORMHØJ MARIA ET AL: "Chimeric Antigen Receptor T Cells Targeting CD79b Show Efficacy in Lymphoma with or without Cotargeting CD19", CLINICAL CANCER RESEARCH, 22 August 2019 (2019-08-22), pages 1 - 20, XP093355296, Retrieved from the Internet <URL:https://aacrjournals.org/clincancerres/article/25/23/7046/82322/Chimeric-Antigen-Receptor-T-Cells-Targeting-CD79b> DOI: 10.1158/1078-0432.CCR-19-1337
- ESQUINAS ESPERANZA ET AL: "Preclinical development of three novel CARs targeting CD79b for the treatment of non-Hodgkin's lymphoma and characterization of the loss of the target antigen", JOURNAL FOR IMMUNOTHERAPY OF CANCER, vol. 12, no. 12, 18 December 2024 (2024-12-18), GB, pages e009485, XP093355124, ISSN: 2051-1426, DOI: 10.1136/jitc-2024-009485

## Description

### Technical field of the invention

The present invention provides therapeutics for the treatment non-Hodgkin lymphoma (NHL). In particular, the present invention provides nucleic acid.

### Background of the invention

Adoptive cellular immunotherapy with autologous T cells modified to express a chimeric antigen receptor (CAR) has improved the treatment of B cell malignancies, including Non-Hodgkin lymphoma (NHL), acute lymphoblastic leukemia (ALL) and multiple myeloma (MM). Specifically, in NHL, there are several approved CAR-T cell products directed to CD19 (CART19) termed axi-cel, tisa-cel, liso-cel, and Tecartus. Axi-cel and tisa-cel have achieved complete responses (CR) of 54% and 53%, respectively, in Diffuse large B cell lymphoma (DLBCL), and TECARTUS of 62% in Mantle cell lymphoma. Moreover, a comparison of Axicel to the standard of care (SOC) as a second line of treatment in NHL showed that CART19 improved the quality of life of patients, and, at a median follow-up of 24.9 months, the median disease-free survival improved to 8.3 months vs 2.0 months. Despite these outstanding responses in relapsed/refractory (R/R) NHL, still, 60% of patients relapse after CART19 therapy due to 1) a loss of expression of the target antigen (CD19) in the tumor cell, which is observed in 27% of relapsed patients after treatment and to 2) a limited persistence of CAR-T cells, which correlates with higher relapse rates and lower PFS. Therefore, it is mandatory to find targets that do not induce the emergence of tumor clones with loss of expression of the target antigen and to increase CAR-T cells' persistence.

Several strategies have been tested to prevent the loss of CD19 after CART19 therapy, including sequential treatment with CARs directed at CD22 or CD20. However, this strategy has caused the loss of the second antigen, CD22, requiring subsequent treatment against a third antigen, CD20. Treatment with tandem or bicistronic CARs targeting two antigens is also promising. Of interest, while tandem CARs against CD19 and CD22 have caused relapses with loss of both antigens, CARs directed at CD19 and CD20 have not caused the loss of the target antigen, suggesting different mechanisms of antigen loss depending on the target. Indeed, treatment with CARs against BCMA (CARTBCMA), which is expressed in NHL, has barely caused loss of BCMA in multiple myeloma (MM).

CAR-T cells directed to BCMA and CD79b can treat NHL. Regarding CD79b, Polatuzumab vedotin (PV) is an antibody-drug conjugate (ADC), which has shown superior disease-free survival to the standard R-CHOP treatment in intermediate-high risk patients with DLBCL. Unfortunately, PV has only demonstrated a short-lived response, suggesting that CAR-based strategies targeting CD79b will yield better clinical results. Moreover, patients who have lost CD19 after CAR-T19 treatment express CD79b, indicating they could be treated with CD79b-based immunotherapies. Therefore, the present invention relates to a new CAR-T therapy against CD79b which overcomes the drawbacks of the therapies Non-Hodgkin Lymphoma mentioned above. US 2021/145878 A1 discloses CARs directed to CD79b that share the same overall scaffold as the CAR CARLY2 of the present invention, namely a CD8 hinge, a CD8 transmembrane domain, a 4-1BB co-stimulatory domain, and a CD3ζ signalling domain.

### Brief description of the invention

The inventors have developed a new CAR-T therapy for NHL based on the antigen CD79b, which consists in three different CARs against CD79b, named CARLY1, CARLY2, and CARLY3.

In a second aspect, the invention relates to a nucleic acid encoding the CAR as disclosed in the present document.

In a third aspect, the invention relates to a cell comprising the nucleic acid according to the second aspect of the invention.

In a fourth aspect, the invention relates to a pharmaceutical composition comprising a plurality of cells according to the third aspect of the invention and a pharmaceutically acceptable carrier or diluent.

In a fifth aspect, the present invention relates to the cell according to the third aspect or the pharmaceutical composition according to the fourth aspect, for use as a medicament. In a further aspect, the present invention relates to the cell according to the third aspect for use in a method of treatment of NHL.

### Definitions

The term "chimeric antigen receptor" or "CAR" refers to a synthetic receptor that targets T cells to a chosen antigen and reprograms T cell function, metabolism and persistence. Similarly, the term "CART" refers to a T cell that comprises a CAR.

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bi-specific antibodies. A typical type of antibody comprises at least two heavy chains ("HC") and two light chains ("LC") interconnected by disulfide bonds. Each "heavy chain" comprises a "heavy chain variable domain" (abbreviated herein as "VH") and a "heavy chain constant domain" (abbreviated herein as "CH"). The heavy chain constant domain typically comprises three constants domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW').

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. The term "effective amount" can be used interchangeably with "effective dose," "therapeutically effective amount," or "therapeutically effective dose".

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration.

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/.

The term "single-chain variable fragment" or "scFv" refers to a fusion protein comprising the variable domains of the heavy chain and light chain of an antibody linked to one another with a peptide linker. The term also includes a disulfide stabilized Fv (dsFv). Methods of stabilizing scFvs with disulfide bonds are disclosed in Reiter et al., 1996. Nat Biotechnol. 14(10):1239-45.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

"Administering" or "administration of" a medicament to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a medicament or provides a patient with a prescription for a drug is administering the drug to the patient.

### Description of the figures

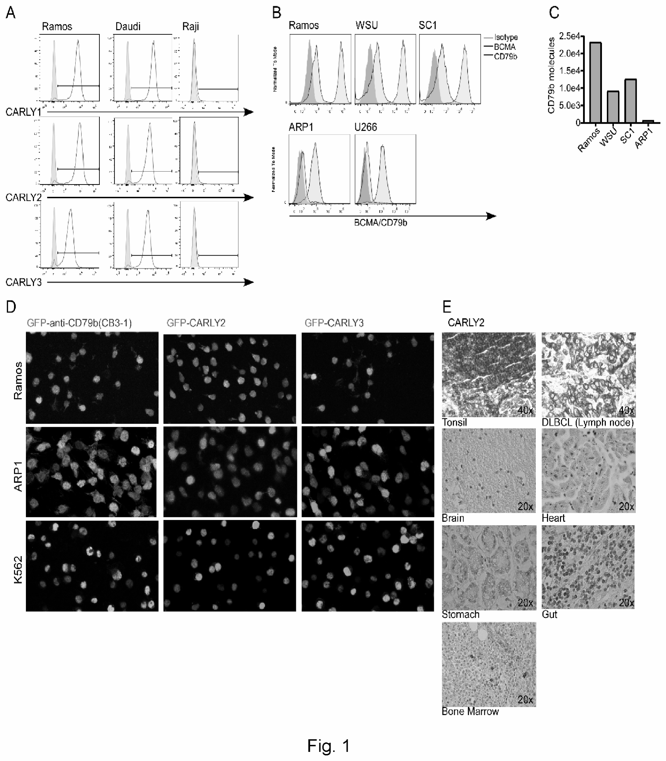

**Figure 1****:** Specificity of the three monoclonals produced against CD79b to design the different CARLYs. A. Flow cytometry staining of NHL (Ramos and Daudi) and T cell lines (Jurkat) using the final clones obtained after hybridoma production of CARLY1, 2, and 3. B. Staining of CD79b of NHL (Ramos, WSU, SC1) and MM (ARP1, U266) cell lines used in all in vitro assays using commercial monoclonals against CD79b (CB3-1 clone) and BCMA (19F2). C. Quantification of the number of CD79b molecules on the surface in NHL and MM cell lines using CB3-1 clone. D. CD79b staining in NHL cells (Ramos), MM cells (ARP1) and a negative control (K562) using a commercial anti-CD79b (clone CB3-1), CARLY2 and CARLY3. Cell lines express GFP (green), and CD79b is shown in red. E. Paraffin embedded tissues stained with CARLY2. Tonsil was used as positive control. DLBCL: Diffuse Large B cell Lymphoma.

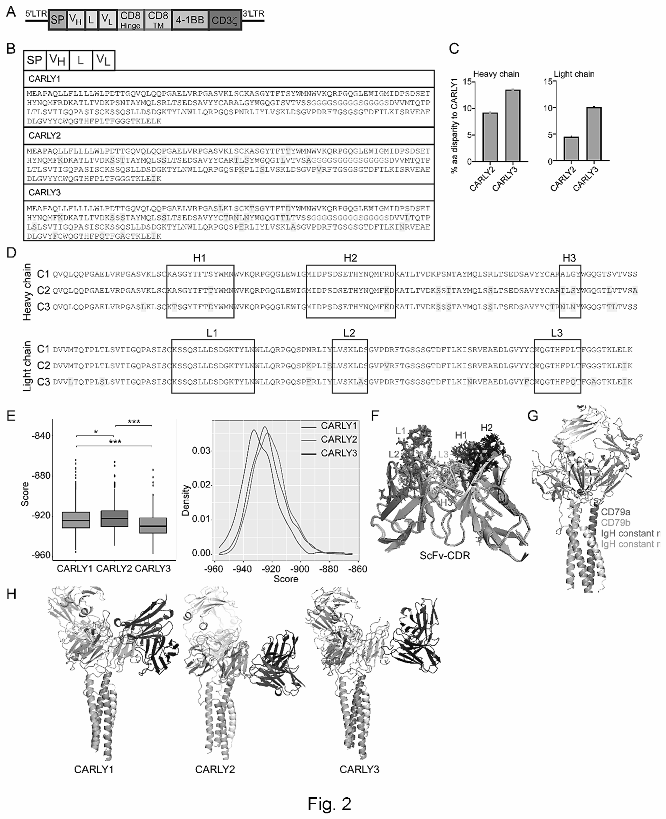

**Figure 2****:** Structural differences in the different scFv of CARLY constructs. A-B. Structure of the CAR constructs, and amino-acid (aa) sequences coding for the signal peptide (SP), variable heavy chain (VH), linker (L) and variable light chain (VL). B: Differences of CARLY2 and CARLY3 compared to CARLY1 are highlighted in yellow. C. % of differences in the aa sequence for the VH and VL sequences shown in B. D. Multiple sequence alignment of the VH and VL of the 3 CARLY constructs. The CDRs for the VH (H1, H2 and H3) and VL (L1, L2 and L3) chains are shown in enclosing rectangles. E. Box plot (left) and normalized distribution (right) showing the predicted binding energy (as Rosetta Scores) for the top 200 docking poses of the complex CD79b-CARLY1,2,3. F. 3D models of the different scFv showing the structural location of the CDRs of the VH (H1, H2, H3) and VL (L1, L2, L3) chains. G. Cryo-EM structure of the heterodimer CD79a/b chains IgH constant mu. Indicated in the black dashed circle is the epitope region of CD79b used to dock the different CARLYs and derive the structural complexes. H. A representative of the 3D docking models of the complex CD79a/CD79b bound to the different scFv.

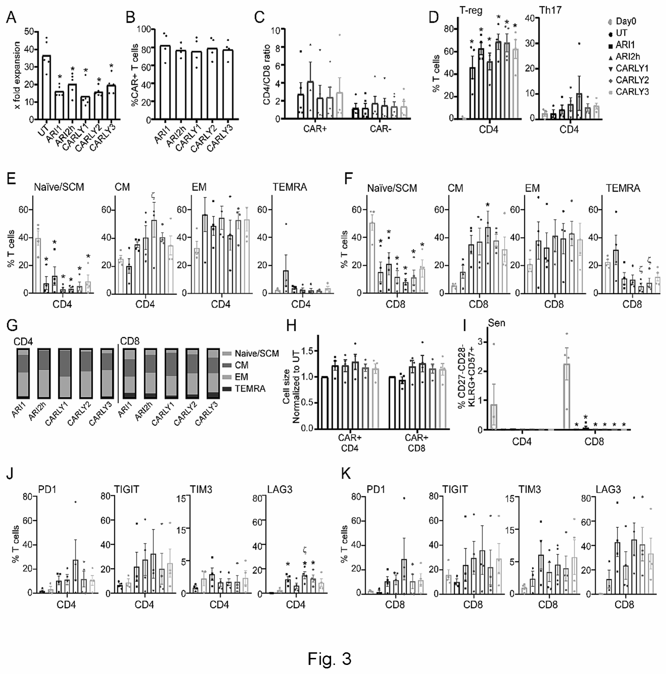

**Figure 3****:** Characterization of CARLY1,2, and 3, ARI1, and ARI2h after their production. A. Fold expansion starting of the different types of T cells starting with 0.25x106 T cells and after 7 days of expansion comparing the different CARs with untransduced T (UT) cells. Statistics are performed comparing vs UT T cells. B-D. Percentage of CARs (B), CD4/CD8 ratio (C) and T-regs and Th17 (D) in the CD4 T cell subset obtained in 4 different donors. E-K. Phenotype characterization of the different CAR products obtained of 4 different donors. Values at day 0 before CAR transduction are shown. E-G. The different T cell differentiation stages (Naïve, Stem cell memory (SCM), central memory (CM), effector memory (EM) terminally differentiated (TEMRA) T cells in CD4 (E) and CD8 (F) are shown. G shows an average of the 4 donors. H-I. T cell size (H) and proportion of senescent CAR+ T cells (I) defined as CD27-CD28-KLRG+CD57+. J-K: Expression of markers related to exhaustion in CD4 (J) and CD8 (K) CAR+ T cells. In A-K statistics were performed comparing vs day 0 (*: p<0.05) or vs UT T cells (ζ: p<0.05).

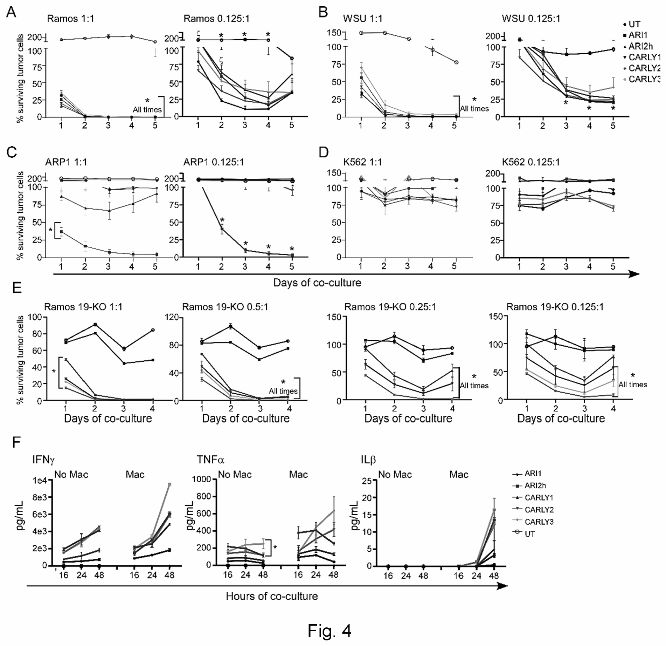

**Figure 4****:** Anti-NHL in vitro activity and inflammatory profile of CARLY cells compared to ARI1 or ARI2h cells: A-D: In vitro cytotoxicity assays of the different types of T cells (Untransduced: UT, and the different CAR-T cells) co-cultured with NHL-Ramos and WSU cells (A and B), MM-ARP1 cells (C) and K562 (D) cells used as negative control. Assays were performed at different Effector:target (E:T) ratios. E. In vitro cytotoxicity assays of the different types of T cells in a model of NHL-Ramos cells with absence of CD19 (19-KO). F. F. IFNγ, TNFα and IL1β of Ramos cells co-cultured with the different CARs in the presence/absence of macrophages at 0.5:1 E:T ratios adding half proportion of macrophages compared to T cells. In A-E statistics were performed comparing vs UT and in F vs ARI1 cells (*: p<0.05 compared to UT T cells, ζ: p<0.05 compared to ARI1).

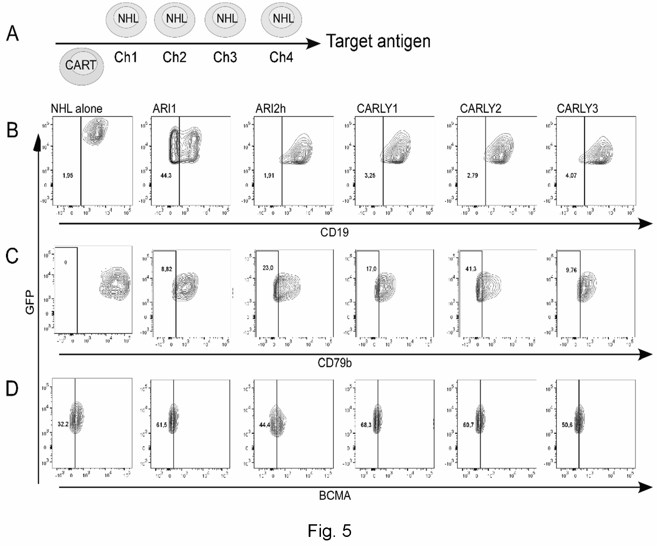

**Figure 5****:** Loss of expression of the target antigen in NHL cells: A: CAR-T cells were consecutively challenged (Ch) to Ramos-NHL cells, and when they were not killed any longer by CAR-T cells, the expression of the target antigen was analyzed. B-D: Flow cytometry plots of Ramos-NHL cells alone and after challenging CAR-T cells four times to NHL cells. CD19, CD79b and BCMA expression was analyzed.

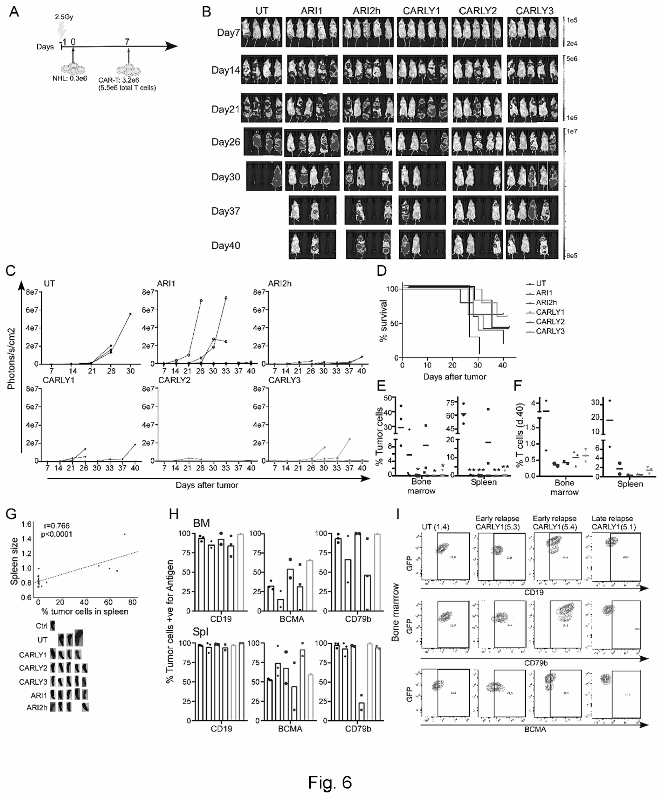

**Figure 6****:** In vivo efficacy of the different CARLY cells compared to ARI1 or ARI2h cells. A. Timing and doses of cells that mice received. B-C. Disease progression followed weekly by bioluminescence. C represents numerical luminescence values of images in B. D. Mice survival of the different groups. E-F: Percentage of tumor cells (E) at final point for each mice and of T cells at day 40 (F) in bone marrow and spleen. G. Correlation analysis (left) of size spleen (right) with the percentage of tumor cells found in the spleen of the different mice. H-I. Analysis of the expression of the target antigen in tumor cells in bone marrow (BM) and Spleen (H). I. Plots showing the expression of target antigens in mice treated with CARLY1 or UT T cells, comparing mice where disease progressed quickly (early relapse, mice 5.3 and 5.4) and mice where disease progressed later (late relapse, mouse 5.1). *: p<0.05 compared to UT T cells.

### Detailed description of the invention

In a first aspect, the present invention provides a chimeric antigen receptor (CAR) comprising an extracellular domain comprising a CD79b targeting-moiety, a transmembrane domain, a co-stimulatory domain and an intracellular signaling domain.

In some embodiments, the CD79b-targeting moiety is an antibody or scFv that specifically binds to CD79b.

### Definition of CARLY 3

In a embodiment of the invention refers to a chimeric antigen receptor (CAR) T-cells that can target CD79b. This embodiment is named as CARLY 3. This CAR comprises a CD79b -targeting moiety, preferably an antibody or scFv, comprises a VH domain, wherein said VH domain comprises:
a heavy chain hypervariable region H1 that comprises the amino acid SEQ ID NO 1: *KTSGYTFTDYWM* ; or
a heavy chain hypervariable region H2 that comprises the amino acid SEQ ID NO 2: *MIDPSDSETHYNQMFKD,* or
a heavy chain hypervariable region H3 that comprises the amino acid SEQ ID NO 3: *NLNY.*

In a preferred embodiment, the CD79b -targeting moiety, preferably an antibody or scFv, comprises a VH domain, wherein said VH domain comprises:
a heavy chain hypervariable region H1 that comprises the amino acid SEQ ID NO 1; and
a heavy chain hypervariable region H2 that comprises the amino acid SEQ ID NO 2, and
a heavy chain hypervariable region H3 that comprises the amino acid SEQ ID NO 3.

In a more preferred embodiment, the CD79b -targeting moiety, preferably an antibody or scFv, preferable is a murine antibody, and more preferred is a murine scFv that comprises a VH domain, wherein said VH domain comprises or consists of SEQ ID NO 4: 
QVQLQQPGAELVRPGASLKLSCKTSGYTFTDYWMNWVKQRPGQGLEWIGMI
DPSDSETHYNQMFKDKATLTVDKSSSTAYMQLSSLTSEDSAVYYCTRNLNYW
GQGTTLTVSS
 or a variant thereof having at least an 85%, 90% 95%, 96%, 97%, 98%, or 99% sequence identity with SEQ ID NO 4,

In another embodiments, the CD79b -targeting moiety, preferably an antibody or scFv, comprises a VL domain, wherein said VL domain comprises:
a heavy chain hypervariable region L1 that comprises the amino acid SEQ ID NO 5: *KSSQSLLDSDGKTYLN;* or
a heavy chain hypervariable region L2 that comprises the amino acid SEQ ID NO 6: *LVSKLAS;* or
a heavy chain hypervariable region L3 that comprises the amino acid SEQ ID NO 7: *WQGTHFPQT.*

In a preferred embodiment, the CD79b -targeting moiety, preferably an antibody or scFv, comprises a VL domain, wherein said VL domain comprises:
a heavy chain hypervariable region L1 that comprises the amino acid SEQ ID NO 5; and
a heavy chain hypervariable region L2 that comprises the amino acid SEQ ID NO:6, and
a heavy chain hypervariable region L3 that comprises the amino acid SEQ ID NO: 7.

In a more preferred embodiment, the CD79b -targeting moiety, preferably an antibody or scFv, preferable is a murine antibody, and more preferred is a murine scFv that comprises a VL domain, wherein said VL domain comprises or consists of SEQ ID NO 8: 
DVVLTQTPLSLSVTIGQPASISCKSSQSLLDSDGKTYLNWLLQRPGQSPERLIY
LVSKLASGVPDRFTGSGSGTDFTLKINRVEAEDLGVYFCWQGTHFPQTFGAG
TKLEIK
 or a variant thereof having at least an 85%, 90% 95%, 96%, 97%, 98%, or 99% sequence identity with SEQ ID NO 8.

In another embodiment, the CD79b -targeting moiety, preferably an antibody or scFv, comprises a VH domain, wherein said VH domain comprises:
a heavy chain hypervariable region H1 that comprises the amino acid SEQ ID NO 1; or
a heavy chain hypervariable region H2 that comprises the amino acid SEQ ID NO 2, or
a heavy chain hypervariable region H3 that comprises the amino acid SEQ ID NO 3;
and a VL domain, wherein said VL domain comprises:
   a heavy chain hypervariable region L1 that comprises the amino acid SEQ ID NO 5; or
   a heavy chain hypervariable region L2 that comprises the amino acid SEQ ID NO 6; or
   a heavy chain hypervariable region L3 that comprises the amino acid SEQ ID NO 7.

In another embodiment, the CD79b -targeting moiety, preferably an antibody or scFv, comprises a VH domain, wherein said VH domain comprises:
a heavy chain hypervariable region H1 that comprises the amino acid SEQ ID NO 1; and
a heavy chain hypervariable region H2 that comprises the amino acid SEQ ID NO 2, and
a heavy chain hypervariable region H3 that comprises the amino acid SEQ ID NO 3;
and a VL domain, wherein said VL domain comprises:
   a heavy chain hypervariable region L1 that comprises the amino acid SEQ ID NO 5; and
   a heavy chain hypervariable region L2 that comprises the amino acid SEQ ID NO 6; and
   a heavy chain hypervariable region L3 that comprises the amino acid SEQ ID NO 7.

In another more preferred embodiment, said CD79b-targeting moiety is humanized antibody or murine antibody. In another more preferred embodiment, said CD79b-targeting moiety is humanized scFv or murine scFv.

In some embodiments, the CD79b -targeting moiety, preferably an antibody or scFv, preferable is a murine antibody, and more preferred is a murine scFv that comprises a VH domain and a VL domain, wherein said VH and VL domain comprises or consists of SEQ ID NO 4 and 8 or a variant of any of these sequences as defined above. Preferably said sequence VH and VL domain comprises or consists of SEQ ID NO 9: 
QVQLQQPGAELVRPGASLKLSCKTSGYTFTDYWMNWVKQRPGQGLEWIGMI
DPSDSETHYNQMFKDKATLTVDKSSSTAYMQLSSLTSEDSAVYYCTRNLNYW
GQGTTLTVSSGGGGSGGGGSGGGGSDVVLTQTPLSLSVTIGQPASISCKSSQ
SLLDSDGKTYLNWLLQRPGQSPERLIYLVSKLASGVPDRFTGSGSGTDFTLKI
NRVEAEDLGVYFCWQGTHFPQTFGAGTKLEIK
 or a variant thereof having at least an 85%, 90% 95%, 96%, 97%, 98%, or 99% sequence identity with SEQ ID NO 9.

It is herein noted that the above mentioned VH and VL domain of SEQ ID NO 9 comprises a linker sequence, in particular SEQ ID NO 24: *GGGGSGGGGSGGGGS.* Other linker sequences might, however, be used.

It is further noted that the VH and VL domain, in particular SEQ ID NO 9, might further comprise a signal Peptide, wherein preferably said signal peptide comprises or consists of SEQ ID NO 23: *MEAPAQLLFLLLLWLPDTTG,*
or a variant thereof having at least an 85%, 90% 95%, 96%, 97%, 98%, or 99% sequence identity with SEQ ID NO 23.

### Definition of CARLY 2

In another embodiment of the invention refers to a chimeric antigen receptor (CAR) T-cells that can target CD79b. This embodiment is named as CARLY 2. This CAR comprises a CD79b -targeting moiety, preferably an antibody or scFv, that comprises a VH domain, wherein said VH domain comprises:
a heavy chain hypervariable region H1 that comprises the amino acid SEQ ID NO 10: *KASGYTFTTYWMN* ; or
a heavy chain hypervariable region H2 that comprises the amino acid SEQ ID NO 2: *MIDPSDSETHYNQMFKD,* or
a heavy chain hypervariable region H3 that comprises the amino acid SEQ ID NO 11: *TLSY.*

In a preferred embodiment, the CD79b -targeting moiety, preferably an antibody or scFv, comprises a VH domain, wherein said VH domain comprises:
a heavy chain hypervariable region H1 that comprises the amino acid SEQ ID NO 10; and
a heavy chain hypervariable region H2 that comprises the amino acid SEQ ID NO 2, and
a heavy chain hypervariable region H3 that comprises the amino acid SEQ ID NO 11.

In a more preferred embodiment, the CD79b -targeting moiety, preferably an antibody or scFv, preferable is a murine antibody, and more preferred is a murine scFv that comprises a VH domain, wherein said VH domain comprises or consists of SEQ ID NO 12: 
QVQLQQPGAELVRPGASVKLSCKASGYTFTTYWMNWVKQRPGQGLEWIGMI
DPSDSETHYNQMFKDKATLTVDKSSTTAYMQLSSLTSEDSAVYYCARTLSYW
GQGTLVTVSA
 or a variant thereof having at least an 85%, 90% 95%, 96%, 97%, 98%, or 99% sequence identity with SEQ ID NO 12.

In another embodiments, the CD79b -targeting moiety, preferably an antibody or scFv, comprises a VL domain, wherein said VL domain comprises:
a heavy chain hypervariable region L1 that comprises the amino acid SEQ ID NO 5: *KSSQSLLDSDGKTYLN*; or
a heavy chain hypervariable region L2 that comprises the amino acid SEQ ID NO 13: *LVSKLDS*; or
a heavy chain hypervariable region L3 that comprises the amino acid SEQ ID NO 14: *WQGTHFPLT.*

In a preferred embodiment, the CD79b -targeting moiety, preferably an antibody or scFv, comprises a VL domain, wherein said VL domain comprises:
a heavy chain hypervariable region L1 that comprises the amino acid SEQ ID NO 5; and
a heavy chain hypervariable region L2 that comprises the amino acid SEQ ID NO13, and
a heavy chain hypervariable region L3 that comprises the amino acid SEQ ID NO 14.

In a more preferred embodiment, the CD79b -targeting moiety, preferably an antibody or scFv, preferable is a murine antibody, and more preferred is a murine scFv, comprises a VL domain, wherein said VL domain comprises or consists of SEQ ID NO 15: 
DVVLTQTPLSLSVTIGQPASISCKSSQSLLDSDGKTYLNWLLQRPGQSPERLIY
LVSKLASGVPDRFTGSGSGTDFTLKINRVEAEDLGVYFCWQGTHFPQTFGAG
TKLEIK
 or a variant thereof having at least an 85%, 90% 95%, 96%, 97%, 98%, or 99% sequence identity with SEQ ID NO 15.

In another embodiment, the CD79b -targeting moiety, preferably an antibody or scFv, comprises a VH domain, wherein said VH domain comprises:
a heavy chain hypervariable region H1 that comprises the amino acid SEQ ID NO 10; or
a heavy chain hypervariable region H2 that comprises the amino acid SEQ ID NO 2, or
a heavy chain hypervariable region H3 that comprises the amino acid SEQ ID NO 11;
and a VL domain, wherein said VL domain comprises:
   a heavy chain hypervariable region L1 that comprises the amino acid SEQ ID NO 5; or
   a heavy chain hypervariable region L2 that comprises the amino acid SEQ ID NO 13; or
   a heavy chain hypervariable region L3 that comprises the amino acid SEQ ID NO 14.

In a more preferred embodiment, the CD79b -targeting moiety, preferably an antibody or scFv that comprises:
a VH domain, wherein said VH domain comprising:
a heavy chain hypervariable region H1 that comprises the amino acid SEQ ID NO 10; and
a heavy chain hypervariable region H2 that comprises the amino acid SEQ ID NO 2, and
a heavy chain hypervariable region H3 that comprises the amino acid SEQ ID NO 11; and
a VL domain, wherein said VL domain comprising:
   a heavy chain hypervariable region L1 that comprises the amino acid SEQ ID NO 5; and
   a heavy chain hypervariable region L2 that comprises the amino acid SEQ ID NO 13; and
   a heavy chain hypervariable region L3 that comprises the amino acid SEQ ID NO 14.

In another more preferred embodiment, said CD79b-targeting moiety is humanized antibody or murine antibody. In another more preferred embodiment, said CD79b-targeting moiety is humanized scFv or murine scFv.

In some embodiments, the CD79b -targeting moiety, preferably an antibody or scFv, preferable is a murine antibody, and more preferred is a murine scFv that comprises a VH domain and a VL domain, wherein said VH and VL domain comprises or consists of SEQ ID NO 12 and 15 or a variant of any of these sequences as defined above. Preferably said sequence VH and VL domain comprises or consists of SEQ ID NO 16: 
QVQLQQPGAELVRPGASVKLSCKASGYTFTTYWMNWVKQRPGQGLEWIGMI
DPSDSETHYNQMFKDKATLTVDKSSTTAYMQLSSLTSEDSAVYYCARTLSYW
GQGTLVTVSAGGGGSGGGGSGGGGSDVVMTQTPLTLSVTIGQPASISCKSS
QSLLDSDGKTYLNWLLQRPGQSPKPLISLVSKLDSGVPVRFTGSGSGTDFTLK
ISRVEAEDLGVYYCWQGTHFPLTFGGGTKLEIK
 or a variant thereof having at least an 85%, 90% 95%, 96%, 97%, 98%, or 99% sequence identity with SEQ ID NO 16.

It is herein noted that the above mentioned VH and VL domain of SEQ ID NO 16 comprises a linker sequence, in particular SEQ ID NO 24: *GGGGSGGGGSGGGGS.* Other linker sequences might, however, be used.

It is further noted that the VH and VL domain, in particular SEQ ID NO 16, might further comprise a signal Peptide, wherein preferably said signal peptide comprises or consists of SEQ ID NO 23: *MEAPAQLLFLLLLWLPDTTG*
or a variant thereof having at least an 85%, 90% 95%, 96%, 97%, 98%, or 99% sequence identity with SEQ ID NO 23.

### Definition of CARLY 1

In another embodiment of the invention refers to a chimeric antigen receptor (CAR) T-cells that can target CD79b. This embodiment is named as CARLY 1. This CAR comprises a CD79b -targeting moiety, preferably an antibody or scFv, that comprises a VH domain, wherein said VH domain comprises:
a heavy chain hypervariable region H1 that comprises the amino acid SEQ ID NO 17: *KASGYTFTSYVVMN* ; or
a heavy chain hypervariable region H2 that comprises the amino acid SEQ ID NO 18: *MIDPSDSETHYNQMFRD,* or
a heavy chain hypervariable region H3 that comprises the amino acid SEQ ID NO 19: *ALGY.*

In a preferred embodiment, the CD79b -targeting moiety, preferably an antibody or scFv, comprises a VH domain, wherein said VH domain comprises:
a heavy chain hypervariable region H1 that comprises the amino acid SEQ ID NO 17; and
a heavy chain hypervariable region H2 that comprises the amino acid SEQ ID NO 18, and
a heavy chain hypervariable region H3 that comprises the amino acid SEQ ID NO 19.

In a more preferred embodiment, the CD79b -targeting moiety, preferably an antibody or scFv, preferable is a murine antibody, and more preferred is a murine scFv, that comprises a VH domain, wherein said VH domain comprises or consists of SEQ ID NO 20: 
QVQLQQPGAELVRPGASVKLSCKASGYTFTSYWMNWVKQRPGQGLEWIGMI
DPSDSETHYNQMFRDKATLTVDKPSNTAYMQLSRLTSEDSAVYYCARALGY
WGQGTSVTVSS
 or a variant thereof having at least an 85%, 90% 95%, 96%, 97%, 98%, or 99% sequence identity with SEQ ID NO 20.

In another embodiments, the CD79b -targeting moiety, preferably an antibody or scFv, comprises a VL domain, wherein said VL domain comprises:
a heavy chain hypervariable region L1 that comprises the amino acid SEQ ID NO 5: *KSSQSLLDSDGKTYLN*; or
a heavy chain hypervariable region L2 that comprises the amino acid SEQ ID NO 13: *LVSKLDS;* or
a heavy chain hypervariable region L3 that comprises the amino acid SEQ ID NO 14: *WQGTHFPLT.*

In a preferred embodiment, the CD79b -targeting moiety, preferably an antibody or scFv, comprises a VL domain, wherein said VL domain comprises:
a heavy chain hypervariable region L1 that comprises the amino acid SEQ ID NO 5; and
a heavy chain hypervariable region L2 that comprises the amino acid SEQ ID NO 13, and
a heavy chain hypervariable region L3 that comprises the amino acid SEQ ID NO 14.

In a more preferred embodiment, the CD79b -targeting moiety, preferably an antibody or scFv, preferable is a murine antibody, and more preferred is a murine scFv that comprises a VL domain, wherein said VL domain comprises or consists of SEQ ID NO 21: 
DVVMTQTPLTLSVTIGQPASISCKSSQSLLDSDGKTYLNWLLQRPGQSPNRLI
YLVSKLDSGVPDRFTGSGSGTDFTLKISRVEAEDLGVYYCWQGTHFPLTFGG
GTKLELK
 or a variant thereof having at least an 85%, 90% 95%, 96%, 97%, 98%, or 99% sequence identity with SEQ ID NO 21.

In another embodiment, the CD79b -targeting moiety, preferably an antibody or scFv, comprises a VH domain, wherein said VH domain comprises:
a heavy chain hypervariable region H1 that comprises the amino acid SEQ ID NO 17; or
a heavy chain hypervariable region H2 that comprises the amino acid SEQ ID NO 18, or
a heavy chain hypervariable region H3 that comprises the amino acid SEQ ID NO 19; and a VL domain, wherein said VL domain comprises:
   a heavy chain hypervariable region L1 that comprises the amino acid SEQ ID NO 5; or
   a heavy chain hypervariable region L2 that comprises the amino acid SEQ ID NO 13; or
   a heavy chain hypervariable region L3 that comprises the amino acid SEQ ID NO 14.

In a more preferred embodiment, the CD79b -targeting moiety, preferably an antibody or scFv that comprises:
a VH domain, wherein said VH domain comprises:
a heavy chain hypervariable region H1 that comprises the amino acid SEQ ID NO 17; and
a heavy chain hypervariable region H2 that comprises the amino acid SEQ ID NO 18, and
a heavy chain hypervariable region H3 that comprises the amino acid SEQ ID NO 19; and
a VL domain, wherein said VL domain comprises:
   a heavy chain hypervariable region L1 that comprises the amino acid SEQ ID NO 5; and
   a heavy chain hypervariable region L2 that comprises the amino acid SEQ ID NO 13; and
   a heavy chain hypervariable region L3 that comprises the amino acid SEQ ID NO 14.

In another more preferred embodiment, said CD79b-targeting moiety is humanized antibody or murine antibody. In another more preferred embodiment, said CD79b-targeting moiety is humanized scFv or murine scFv.

In some embodiments, the CD79b -targeting moiety, preferably an antibody or scFv, preferable is a murine antibody, and more preferred is a murine scFv that comprises a VH domain and a VL domain, wherein said VH and VL domain comprises or consists of SEQ ID NO 20 and 21 or a variant of any of these sequences as defined above. Preferably said sequence VH and VL domain comprises or consists of SEQ ID NO 22: 
QVQLQQPGAELVRPGASVKLSCKASGYTFTSYWMNWVKQRPGQGLEWIGMI
DPSDSETHYNQMFRDKATLTVDKPSNTAYMQLSRLTSEDSAVYYCARALGY
WGQGTSVTVSSGGGGSGGGGSGGGGSDVVMTQTPLTLSVTIGQPASISCKS
SQSLLDSDGKTYLNWLLQRPGQSPNRLIYLVSKLDSGVPDRFTGSGSGTDFT
LKISRVEAEDLGVYYCWQGTHFPLTFGGGTKLELK
 or a variant thereof having at least an 85%, 90% 95%, 96%, 97%, 98%, or 99% sequence identity with SEQ ID NO 22.

It is herein noted that the above mentioned VH and VL domain of SEQ ID NO 22 comprises a linker sequence, in particular SEQ ID NO 24: *GGGGSGGGGSGGGGS.* Other linker sequences might, however, be used.

It is further noted that the VL and VH domain, in particular SEQ ID NO 22, might further comprise a signal Peptide, wherein preferably said signal peptide comprises or consists of SEQ ID NO 23: *MEAPAQLLFLLLLWLPDTTG*
or a variant thereof having at least an 85%, 90% 95%, 96%, 97%, 98%, or 99% sequence identity with SEQ ID NO 23.

In a embodiment of the invention, the transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, or CD154. Preferably, the transmembrane regions comprise at least the transmembrane region(s) CD8a.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has at least an 85%, 90% 95%, 96%, 97%, 98%, or 99% sequence identity. Preferably, the said transmembrane domain comprises or consists of at least the transmembrane domain of CD8a.

In some embodiments, the transmembrane domain comprises or consists of the transmembrane domain of CD8a or a variant thereof, wherein the variant thereof has at least 85%, 90% 95%, 96%, 97%, 98%, or 99% sequence identity.

In particular, in some embodiments, the transmembrane domain comprises or consists of SEQ ID NO: 26 or a variant thereof having at least an 85%, 90% 95%, 96%, 97%, 98%, or 99% sequence identity with SEQ ID NO 26.

Transmembrane domain derived from CD8a (SEQ ID NO 26: *YIWAPLAGTCGVLLLSLVITLYC).*

In some embodiments the domain derived from CD8a is bound directly to a CD8 hinge, preferably to SEQ ID NO 25:
*TTTPAPRPPTPAPTIASQPLSLRPEACRPAAGGAVHTRGLDFACDI*
or to a variant thereof having at least an 85%, 90% 95%, 96%, 97%, 98%, or 99% sequence identity with SEQ ID NO 25.

Therefore, in further some embodiments the transmembrane domain further comprises a CD8 hinge and comprises or consists of SEQ ID NO: 27 
TTTPAPRPPTPAPTIASQPLSLRPEACRPAAGGAVHTRGLDFACDIYIWAPLAG
TCGVLLLSLVITLYC
 or a variant thereof having at least an 85%, 90% 95%, 96%, 97%, 98%, or 99% sequence identity with SEQ ID NO 27.

It is noted, that the CAR of the invention must further comprise a costimulatory signaling domain. In some embodiments, the costimulatory signaling domain comprises the intracellular domain of 4-1BB or a variant thereof, wherein the variant thereof has at least an 85%, 90%, 95%, 96%, 97%, 98%, or 99% sequence identity.

In some embodiments, the costimulatory signaling domain comprises or consists of the intracellular domain of 4-1BB or a variant thereof, wherein the variant thereof has at least an 85%, 90% 95%, 96%, 97%, 98%, or 99% sequence identity.

In some embodiments, the costimulatory signaling domain comprises or consists of SEQ ID NO: 28 or to a variant thereof having at least an 85%, 90% 95%, 96%, 97%, 98%, or 99% sequence identity with SEQ ID NO 28. It is noted that the costimulatory signaling domain derived from 4-1BB is herein represented by SEQ ID NO: transmembrane domain bound:
*KRGRKKLLYIFKQPFMRPVQTTQEEDGCSCRFPEEEEGGCEL*

It is noted that the CAR of the invention must further comprise a intracellular signaling domain. The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises or consists of the intracellular domain of CD3ζ or a variant thereof, wherein the variant thereof has at least an 85%, 90% 95%, 96%, 97%, 98%, or 99% sequence identity.

In some embodiments, the intracellular signaling domain comprises SEQ ID NO: 29, or a variant thereof having at least an 85%, 90% 95%, 96%, 97%, 98%, or 99% sequence identity with SEQ ID NO 29. It is noted that SEQ ID NO: 29 is represented by the following sequence:

RVKFSRSADAPAYQQGQNQLYNELNLGRREEYDVLDKRRGRDPEMGGKP
QRRKNPQEGLYNELQKDKMAEAYSEIGMKGERRRGKGHDGLYQGLSTATK
DTYDALHMQALPPR

In a second aspect, the invention relates to a nucleic acid encoding the CAR as disclosed in the present document. The nucleic acid sequence that encodes the chimeric receptor links together a number of modular components that can be excised and replaced with other components in order to customize the chimeric receptor for efficient T cell activation and recognition of CD79b.

It is noted, in a preferred embodiment, the nucleotide sequence of the CD79b -targeting moiety, preferably the VH domain and VL domain with the linker, of the embodiment named as CARLY 3 according to the present invention might preferably comprise or consists of SEQ ID NO 30:

CAGGTCCAGCTTCAGCAGCCGGGTGCTGAATTGGTAAGACCTGGCGCGTCTCTTAAACTGAG
TTGTAAAACGTCTGGATATACCTTCACCGACTACTGGATGAACTGGGTTAAACAAGACCGG
GACAGGGTTTGGAATGGATTGGTATGATAGATCCTAGTGATTCAGAAACTCACTATAATCAA
ATGTTCAAAGACAAAGCTACACTCACCGTTGACAAATCTTCTTCTACGGCTTATATGCAGCT
CAGCTCCCTCACCAGCGAGGACAGTGCCGTTTATTACTGCACTCGCAACTTGAATTACTGGG
GACAGGGAACGACTTTGACCGTCAGTAGCGGCGGCGGGGATCAGGCGGCGGTGGTTCTGGC
GGGGGTGGCTCCGATGTGGTTTTGACTCAAACACCACTTTCACTTAGTGTTACTATTGGTCA
ACCGGCCAGCATATCTTGTAAGTCTAGCCAAAGTTTGCTGGATTCCGACGGAAAACATACT
TGAACTGGCTGCTCCAACGCCCAGGACAGTCCCCTGAAAGATTGATATACCTCGTATCTAAA
CTGGCAAGCGGAGTGCCAGATCGCTTTACCGGGAGCGGATCTGGTACTGATTTCACTCTGAA
AATCAATAGAGTTGAAGCCGAGGACCTCGGCGTATATTTTTGCTGGCAGGGTACCCACTTTC
CGCAAACATTTGGCGCTGGGACGAAGCTGGAAATTAAA

It is noted, in a preferred embodiment, the nucleotide sequence of the CD79b -targeting moiety, preferably the VH domain and VL domain with the linker, of the embodiment named as CARLY 2 according to the present invention might preferably comprise or consists of SEQ ID NO 31:

CAAGTGCAACTGCAGCAGCCCGGGGCGGAACTGGTTCGGCCCGGGGCGAGTGTGAAACTTAG
CTGCAAAGCTTCCGGATACACATTCACTACGTACTGGATGAACTGGGTTAAACAAGGCCTG
GGCAGGGCCTCGAGTGGATCGGATGATAGACCCGTCTGATTCTGAAACCCATTATAACCAA
ATGTTCAAAGACAAGGCGACGTTGACAGTAGATAAGTCTTCTACGACTGCCTACATGCAATT
GAGCAGTTTGACAAGTGAAGATTCAGCTGTGTATTACTGTGCGAGAACCCTGTCATACTGGG
GCCAAGGTACTCTCGTAACCGTTAGTGCCGGAGGAGGCGGCTCTGGAGGAGGTGGATCCGGT
GGTGGTGGTTCAGACGTAGTTATGACACAAACTCCTTTGACACTCAGCGTAACGATCGGACA
GCCGGCTTCAATTAGTTGCAAAAGTAGTCAGTCTTTGCTCGACTCAGATGGCAAGACGTATC
TGAACTGGCTTCTGCAGCGCCCCGGACAGAGCCCTAAACCACTGATATCCCTGGTCTCTAAG
CTTGATAGTGGCGTTCCTGTGCGCTTCACGGGGAGCGGTAGCGGAACCGATTTCACACTTAA
GATTTCTAGAGTGGAAGCAGAGGACTTGGGCGTATATTATTGCTGGCAGGGGACACACTTCC
CTCTGACTTTCGGAGGCGGAACTAAACTCGAAATAAAA

It is noted, in a preferred embodiment, the nucleotide sequence of the CD79b -targeting moiety, preferably the VH domain and VL domain with the linker, of the embodiment named as CARLY 1 according to the present invention might preferably comprise or consists of SEQ ID NO 32:

CAGGTTCAACTGCAACAACCAGGAGCGGAGCTGGTCCGACCGGGGCTAGTGTCAAATTGTC
ATGTAAGGCAAGTGGATATACTTTTACAAGCTATTGGATGAATTGGGTAAAGCAGCGACCCG
GGCAAGGGCTTGAGTGGATTGGCATGATTGACCCTAGTGACTCAGAGACCCACTACAACCAG
ATGTTCAGAGACAAGGCAACCTTGACAGTTGACAAGCCAAGTAATACTGCGTATATGCAGTT
GAGTCGACTTACATCAGAGGACTCTGCAGTGTATTATTGCGCTAGGGCACTCGGCTATTGGG
GACAAGGGACCTCCGTCACCGTATCTTCCGGAGGTGGTGGATCTGGAGGAGGCGGATCTGGT

GGCGGGGGTCCGACGTCGTTATGACCCAGACTCCACTTACACTCTCCGTTACGATTGGACA
GCCCGCTTCAATAAGTTGTAAAGTTCCCAGTCACTGCTCGACAGCGATGGTAAGACGTACC
TCAACTGGTTGCTCCAAAGACCCGGCCAAAGCCCCAACCGCCTGATCTATCTCGTCAGTAAA
TTGGATTCCGGTGTCCCTGACAGGTTCACGGGTTCTGGTAGCGGACAGACTTTACGCTCAA
AATATCTAGAGTTGAAGCAGAAGATCTTGGAGTTTATTATTGTTGGCAAGGACGCACTTTC
CGCTTACGTTCGGGGGAGGGACTAAACTTGAACTCAAA

In a third aspect, the present invention provides a cell comprising the nucleic acid of the present invention and/or the CAR of the present invention. In some embodiments, the cell is a T-cell, referred to as a CART.

In some embodiments, the cell is a naïve T cell, memory stem T cell or central memory T cell. It is currently thought that these cells are better suited for adaptive immunotherapy (see Rivière & Sadelain, 2017. Mol Ther. 25(5):1117-1124).

In some embodiments, the cell is an autologous T cell. The term "autologous cell" refers to a cell obtained from the same patient that is to be treated using any one of the methods of the present invention.

In some embodiments, the cell is an allo-tolerant T cell. The term "all-tolerant cell" refers to a cell that has been engineered to decrease the risk of a Graft-versus-host disease response. In some embodiments, this is achieved by genomic editing-mediated deletion of TCR and/or β2-microglobulin15,19. Allo-tolerant cells are known in the art (see section of allogeneic T cells in Rivière & Sadelain, 2017. Mol Ther. 25(5):1117-1124).

In some embodiments, the cell is a lymphoid precursor, embryonic stem cell or an induced pluripotent stem cell with the capacity to differentiate into a mature T cell (see Rivière & Sadelain, 2017. Mol Ther. 25(5):1117-1124).

In a fourth aspect, the invention relates to a pharmaceutical composition comprising a plurality of cells according to the third aspect of the invention and a pharmaceutically acceptable carrier or diluent.

A pharmaceutical composition as described herein may also contain other substances. These substances include, but are not limited to, cryoprotectants, surfactants, antioxidants, and stabilizing agents. The term "cryoprotectant" as used herein, includes agents, which provide stability to the CARTs against freezing-induced stresses. Nonlimiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

In a fifth aspect, the present invention relates to the cell according to the third aspect or the pharmaceutical composition according to the fourth aspect, for use as a medicament.

In one aspect, the present invention provides a cell according to the present invention or a pharmaceutical composition according to the present invention for use in a method of treatment of NHL.

In another aspect, the present invention provides a method of treating Non- Hodgkin Lynphoma comprising administering the cell of the present invention or the pharmaceutical composition of the present invention to a patient in need thereof.

In some embodiments, the patient is administered a therapeutically effective amount of cells.

In some embodiments, the cell or pharmaceutical composition is administered is administered intravenously, intraperitoneally, into the bone marrow, into the lymph node, and /or into cerebrospinal fluid.

The following examples serve to illustrate the present invention The scope of protection is defined by the appended claims.

### Examples

### Material and methods

Ethics statement: Research involving human materials was approved by the Clinical Research Ethical Committee (Fundación Jiménez Diaz, Madrid, Spain). Peripheral blood T cells were obtained from healthy donors after informed consent. All animal work was performed with approval from the Animal Research Ethical Committee (Fundación Jiménez Diaz, Madrid, Spain).

Structural modelling of CD79b-CARLY(1,2,3) scFv complexes: The structural models of the CD79b and CARLY(1,2,3) scFv complexes were obtained using data-driven docking as follows. The structure for CD79a in complex with CD79b and IgHM constant mu chain, identification code 7xq8 (24), was taken for the PDB databank (25). The structure of CARLY(1,2,3) scFvs were derived by homology modelling using M4T (26). The structure of CD79b in complex with CD79a and IgHM constant mu chain was used to identify the epitope of CD79b for docking (next). The initial docking conformations were inferred using PatchDock (27) by selecting the complementarity-determining regions (CDRs) of CARLY(1,2,3) scFvs and the predicted epitope of CD79b as docking interfaces. The surface segmentation parameters for CD79b and CARLY(1,2,3) scFvs were set to default except for the hot spot filter type that was set to antigen and antibody, respectively. The resulting docking conformation were optimized and minimized using the SnugDock application (28) within the Rosetta suite (29) with default parameters except refine_outer_cycles, max_inner_cycles and -h3_filter set to 2, 20 and true, respectively. Finally, the resulting minimized complexes were sorted by Rosetta global score and the top 200 poses were visually inspected.

Donors of immune cells: peripheral blood T cells and monocytes were obtained from buffy coats from healthy donors obtained at "Centro de Transfusiones de Madrid" after obtaining informed consent and being approved by the BST.

Tumor cell lines: Ramos, WSU, SC1, and K562 cell lines were purchased from American Tissue Culture Collection (Manassas, Virginia, USA). ARP1 cell line was kindly provided by Multiple Myeloma Research Center (Little Rock, Arkansas, USA) under MTA. All tumor cell lines were cultured in RPMI with 10% fetal bovine serum (FBS) and 1% penicillin/streptomycin (Pen/Strep). Tumor MM cell lines were modified to express GFP-FireFlyLuciferase (GFP-FFLuc) using the plasmids pLV-MSCV_Luc-T2A-GFP (kindly provided by Amer Najjar) coding for GFP-FFLuc, and pMD2.G and psPAX2 coding for VSV-G and gag/pol, respectively. Mycoplasma testing in cell lines was performed every two months.

Lentivirus production for CAR-T cells: All genes coding the different CARLYs were inserted in a pCCL plasmid with EF1a promoter. ARI0001 (from now on ARI1) was provided by Manel Juan (Hospital Clinic of Barcelona), AR10002h (from now on ARI2h) had been produced previously by the authors (23,30). Lentiviral particles were obtained with packaging plasmids pMDLg-pRRE, pRSV-Rev, and envelope plasmid VSV. Production of virus particles to transduce T cells was done in HEK293T cells using the same protocol previously described by us, using NaCl and JetPei, and Lenti-X Concentrator (ClonTech) to collect virus supernatants (30).

CAR manufacturing: T cells were obtained from buffy coats by Ficoll and magnetic T cell depletion (Miltenyi Biotec). T cells were expanded in Click's media (50% RPMI, 50% Click's (Irvine Scientific), 5% human serum, 1% Pen/Strep), activated with Dynabeads Human T-Activator CD3/CD28 (Thermo Fisher Scientific) and IL-2 (100 IU/mL) every other day. Experiments were performed after 8-10 days of T cell expansion. After 48 hours of T cell expansion, viral transduction was performed at a MOI of 10.

Macrophage differentiation: After performing Ficoll, monocytes were isolated with RosetteSep Human Monocyte Enrichment Cocktail (Stem Cell Technologies) and macrophages were differentiated from monocytes after one week in RPMI 10% FBS and 0.1mg/ml M-CSF (Thermo Fisher Scientific).

In vivo models: Immunodeficient NSG mice were purchased from The Jackson Laboratory. Mice were of the same sex and 8-12 weeks old. Mice were irradiated on day -1 at a dose of 2.25 Gy in a biological radiator with two sources of 137 Cs (J.L. Shepherd, model MARK 1, 30 serial number 1199) and received intravenous Ramos-GFP-FFLuc tumor cells on day 0. Seven days later, mice received T cells at the dose indicated in each experiment. The disease was followed weekly by bioluminescence, as previously described (30). Bone marrow (BM) and spleen were harvested when mice were euthanized to analyze the presence of CAR-T cells and tumor cells by flow cytometry.

Cytotoxicity in vitro assays: Luciferase killing assays were performed by co-culturing tumor cells expressing GFP-FFLuc with effector cells in white 96-well plates. D-luciferin (8 □g/mL) was added at the time of plating cells. Bioluminescence was read every day in a Synergy HT Plate Reader (BioTek). The percentage of live tumor cells was calculated as (luminescence of sample/luminescence of tumor cells alone) ×100.

Cytokine production: Interferon gamma (IFNγ), tumor necrosis factor alpha (TNFα) and Interleukin 1-beta (IL-1β were quantified by ELISA (ELISA MAX Deluxe Set, Biolegend) following the manufacturer's protocol.

Confocal microscopy: Ramos, ARP1 and K562 cell lines modified to express GFP were plated in Poly-D-Lysine coated slides and stained for CD79b using in parallel the monoclonal CB3-1, CARLY2 and CARLY3. Anti-mouse IgG-Alexa647 was used as the secondary antibody. Images were acquired using a Leica SP5 microscope. 405, 488 and 633 lasers were used for excitation.

Challenges: Ramos-GFP-FFLuc cells were co-cultured with CART cells at an effector:target ratio 0.5:1. Every day, luminescence was read to assess tumor cell survival. When tumor cells had been killed, a new challenge to tumor cells was performed.

Flow cytometry: CAR expression for ARI2h was detected with either a recombinant BCMA-Fc protein (Enzo Life Sciences) and an anti-human IgG Fc-BV-421 (Biolegend, clone: M1310G05). For the other CARs, a Biotin-SP AffiniPure Goat Anti-Mouse IgG, F(ab')₂ (Jackson Immunoresearch), and Streptavidin Super Bright 436 Conjugate (Thermo Fisher Scientific) were used. For T cell phenotyping, PD-1-APC (clone: J105), TIM-3-FITC (clone: F38-2E2), TIGIT-PerCP-Cy5.5 (clone: A15153G), LAG-3-PE (clone: 11C3C65), CXCR3-Alexa-Fluor 488 (Clone 1C6/CXCR3), CCR7-PerCP-Cy5.5 (clone: 150503), CD45RA-APC (clone: HI100), CD27-PE (clone: M-T271), CD28-FITC (Clone CD28.2), CD3-PE (clone SK7), CD4-APC-H7 (clone L200) and CD8-PE-Cy7 (clone: RPA-T8) antibodies were used. For T cell determination in BM or spleen at in vivo end-point, mouse FcR blocking reagent (Miltenyi) was used. All experiments were read on a FACS Canto II (BD Biosciences) and analyzed with FlowJo software (Tree Star, Eugene, Oregon, USA).

Graphs and statistical analysis: Data and statistical analysis were presented and performed using GraphPad Prism software v8.0.1. Anova Test was conducted to compare between groups.

### Results

The three monoclonals produced against CD79b are highly specific recognizing NHL cells.

An external service produced three monoclonal murine antibodies against CD79b. We first confirmed their reactivity against NHL cell lines (Ramos and Daudi), and a T cell line (Raji) by flow cytometry, ensuring that they reacted against B cells and not against T cells (Figure 1A). These monoclonals were named CARLY (CAR for Lymphoma) 1, 2 and 3. In parallel, NHL (Ramos, SC1, and WSU) and multiple MM (ARP1 and U266) cell lines that were used for all in vitro studies were stained for CD79b using a commercial anti-CD79b and BCMA antibodies (Ab). NHL cells presented high and low staining for CD79b and BCMA, respectively. MM cells lines showed high levels of BCMA and almost undetectable staining for CD79b (Figure 1B). By measuring the number of CD79b molecules on the cell membrane, we confirmed that NHL cells (Ramos, WSU, and SC1) had a very high number of CD79b molecules on the membrane, being Ramos, the cell line with the highest value. In addition, ARP1-MM cell lines presented an almost undetectable number of CD79b molecules on the membrane (Figure 1C). As CD79b staining in cell lines (Figure 1B and 1C) had been performed with a commercial Ab (clone CB3-1), we compared the CD79b pattern obtained with our monoclonals (CARLY2 and CARLY3) on NHL cells, MM cells and K562, a chronic myeloid leukemia cell line, that does not express B cell markers. We noticed that CARLY2 and CARLY3 only stained Ramos cell line, whereas CB3-1 stained ARP1 MM cell lines with a very clear membrane pattern, and also in a lower degree, K562 cells (Figure 1D), indicating the high specificity of CARLY2 and CARLY3 towards NHL cells. In addition, to have an idea of the possible on-target, off-tumor toxicity that our future CARs could have, we stained paraffin-embedded (FFPE) tissue samples of different healthy tissues and lymph node tissues of DLBCL patients. CARLY2 stained the positive controls (Tonsil) and lymph nodes from DLBCL. No staining was observed in other healthy tissues (brain, heart and stomach), and some staining corresponding to B cells in BM and the gut was detected (Figure 1E).

The scFvs coding for CARLY1 and CARLY2 have higher structural homologies being CARLY3 the most different.

Based on the high specificity of our monoclonal antibodies towards CD79b, we sequenced the three scFvs to design our CAR constructs afterward. The three CARs (CARLY1, 2, and 3) were 2nd generation CAR-T cells with 4-1BB as co-stimulatory domain, CD8a as hinge and transmembrane domains, they had the same signal peptide, and heavy and light chains were separated by 15-mer linker (GGGGSx3) (Figure 2A-2B). Sequences were aligned, and the differences in the number of amino acids (aa) were analyzed by comparing CARLY2 and CARLY3 vs CARLY1. The percentage of aa disparity compared to CARLY1 was higher for CARLY3 in both the heavy and light chains (Figure 2B and 2C). This higher disparity for CARLY3 was maintained in the Complementarity Determining Regions (CDRs) responsible for recognizing the tumor epitope (Figure 2D). To have additional information, we performed predictive binding affinity models towards CD79b. Binding energy was lower for CARLY3, then CARLY2, and then CARLY1 (Figure 2E), indicating that CARLY3 might have a higher binding affinity, and CARLY1 and 2 would have a similar binding affinity. Structural predictive models of the different scFvs complexes showed some structural differences in the location of the CDRs (Figure 2F). Structural predictive models also predicted the exposed area of CD79b when complexed with CD79a and with IgH constant mu that the scFv would bind (Figure 2G and 2H).

Manufacturing of CARLY1, 2, and 3 does not cause high differences in the T cell phenotype among the three CARLYs.

We compared differences in the CAR-T cell product after manufacturing, comparing the three CARLYs, with untransduced T (UT) cells and with a CART19 (ARI1) and a CARTBCMA (ARI2h). All the CARs reached similar expansion levels, reaching all of them lower expansion levels than UT T cells. CARLY1 showed a trend for lower expansion than the other CARs (Figure 3A). For all CARs, the efficacy of CAR transduction was higher than 80% (Figure 3B). The CD4 and CD8 T cell proportions also did not vary significantly between CARs and the UT T cells. However, in the final product, CAR+ T cells contained a higher proportion of CD4 T cells than CAR- T cells (Figure 3C). We analyzed the ratio of T regs (CD4+, CD25+CD127-) (31) and Th17 cells in the CD4 population. As T cells expanded with the addition of IL2, T-regs increased significantly from day 0 to day 7, reaching over 50% of CD4 T cells. Th17 subpopulation did not vary from day 0 and remained at a low profile, hovering around 15% of the cells analyzed. No differences were observed among the different CARs (Figure 3D).

Analysis of differentiation stages of T cells did not show differences among the different CARs, despite the high variability observed in the different donors. As expected, naïve and stem cell memory (SCM) T cells decreased significantly after CAR manufacturing for all groups of CD4 and CD8 T cells, as they differentiated into central memory (CM) and effector memory (EM) T cell subsets (Figure 2E and 2F). CARLY1 showed a trend for a higher loss of naïve and SCM T cells during expansion, which translated in a significant increase of CM CD4 T cells compared to UT T cells, and increased CD8 CM T cells from day 0 (Figure 3E and 3F). Moreover, terminally differentiated (TEMRA) CD8 T cells were decreased in CARLY1 and CARLY2 compared to UT T cells (Figure 3E-3G).

Senescent T cells can be defined by a loss of CD28 and CD27 and acquisition of CD57 and KLRG1 (32). Increasing cell size might also indicate senescent stages (33,34). No differences were observed in cell size among the different groups of T cells after manufacturing (Figure 3H). Of interest, on day 0, there was a higher proportion of CD8 senescent T cells compared to CD4 T cells, which dropped significantly to less than 1% in all CAR groups following CAR manufacturing and showed no differences in the different T cell products (Figure 31).

The analysis of T cell exhaustion did not show differences between the different CARs (Figure 3G and 3K). Despite the variability among different donors, compared to day 0, CD4 Lag3+ T cells increased in CARLY1, CARLY2, and ARI1 cells. Moreover, CARLY1 also had a higher proportion of CD4 Lag3+ T cells than UT T cells after manufacturing (Figure 3J).

Anti-NHL in vitro efficacy and inflammatory profile of CARLY cells compared to ARI1 or ARI2h cells.

The anti-NHL activity of the different CARLY cells was compared to that of UT-T cells, ARI1 and ARI2h cells. Cytotoxicity assays at different E:T ratios showed that, as expected, all CARs killed NHL cells (Ramos and WSU) with high efficacy at 1:1 E:T ratio. However, at 0.125:1 ratio, we could observe differences. Specifically, at 24h and 48h, ARI1 presented higher cytotoxicity than ARI2h, CARLY1 and CARLY2 (Figure 4A). For WSU-NHL cell line, there were no significant differences between any CAR-T at any ratio, although we could see that at late time-points, CARLY3 maintained a slightly lower killing at low ratios (0.25:1) (Figure 4B). As ARP1 cells do not express CD19 and CD79b antigens, they were only eliminated by ARI2h cells. However, at 1:1 ratio, we observed some ARP1 killing by CARLY1 cells, although this killing was lost when we lowered the ratio (Figure 4C). No killing was observed for any CAR-T in the K562 line, used as a negative control (Figure 4D).

Moreover, to mimic a model of CD19- relapse, we exposed the different CARs to NHL Ramos cells where CD19 had been removed by CrisprCas9 (Ramos 19-KO). As expected, UT T cells and ARI1 cells did not remove NHL cells, whereas all CARLY cells and ARI2h cells killed them at different E:T ratios (Figure 4E).

The pro-inflammatory profile of each CAR was compared by analyzing cytokine production after co-culture with NHL Ramos cells in the presence/absence of macrophages. As expected IFNγ, TNFα , and IL1β production was higher in the presence of macrophages (Figure 4F). ARI1 cells showed the most minor cytokine release. In macrophage-free, the three CARLYs had similar and higher IFNγ production than the other CAR-Ts. TNFα production usually occurs very fast and decays over time (30). Without macrophages, we confirmed this TNFα pattern for all CARs but for CARLY3, whose production started later and was still increasing at 48h. In the presence of macrophages, CARLY2 showed the same trend (Figure 4F). This pattern might indicate either a slower kinetics or a higher pro-inflammatory profile. IL1β as expected, was not detected in the absence of macrophages, and with macrophages, ARI1 and CARLY1 induced the lowest IL1β production.

CARLY cells in vitro do not induce loss of expression of the target antigen in NHL cells.

Loss of expression of the target antigen (CD19, CD79b and BCMA) was analyzed after exposing consecutively CAR-T cells to tumor cells in different challenges and looking at target antigen expression when CAR-T cells stopped killing (Figure 5A). We confirmed that ARI1 induced CD19 loss on tumor cells, and none of the other CARTs did (Figure 5B). Of interest, CD79b reduced its expression after CAR exposure for all the CARs. However, a clear population with loss of CD79b was not observed (Figure 5C). BCMA, expressed at very low levels in resting NHL cells, did not vary after treatment with any CARs (Figure 5D).

CARLY2 and CARLY3 cells present the highest in vivo efficacy in a model of NHL.

The anti-NHL activity of all CARs was compared in vivo in a model of NHL where mice received CAR-T cells seven days after the tumor cells (Figure 6A). Mice treated with UT T cells could not avoid disease progression, whereas all CAR-T cells avoided disease progression in a higher or lower degree (Figure 6B). Specifically, mice treated with any CAR reached a higher survival than mice treated with UT T cells. However, mice treated with CARLY2 and CARLY3 reached the longest survival. CARLY1 presented the shortest survival, followed by ARI1 and ARI2h (Figure 6D).

At final point, flow cytometry analysis of GFP+ tumor cells showed that UT mice had the highest engraftment of tumor cells in BM and spleen, being more abundant in the spleen than in BM (Figure 6E). Analysis of the different treatments showed that ARI1 and CARLY1 conferred worse protection than CARLY2, CARLY3 and ARI2h. Specifically, ARI1 and CARLY1 showed similar tumor engraftment to UT T cells in BM, showing this pattern also CARLY1 in the spleen (Figure 6E). Analysis of CD3 T cells on the surviving mice on day 40 indicated that they were present in a very low percentage for all groups of CARs, and even though some differences were observed, these were not significant (Figure 6F). In addition, we observed that when the disease was out of control, mice developed splenomegaly that correlated with the presence of tumor cells in the spleen (Figure 6G).

Last, we analyzed the expression of the target antigen (CD19, BCMA, CD79b) in tumor cells. Considering the low number of samples with the presence of tumor cells to analyze, no significant findings were observed. However, we noticed some differences among the treatments. For CD19 there wasn't a drastic loss as previously observed in vitro (Figure 6H). For BCMA and CD79b, mice treated with CARLY1 where the disease progressed at late time points (late relapse) had lost the expression of CD79b, and a decrease in BCMA was observed, a finding not observed in cases of early relapse (Figure 61).

## Claims

1. A chimeric antigen receptor (CAR) comprising a CD79b -targeting moiety, that comprises:
• a VH domain, wherein said VH domain comprises:
∘ a heavy chain hypervariable region H1 that comprises the amino acid SEQ ID NO 10; and
∘ a heavy chain hypervariable region H2 that comprises the amino acid SEQ ID NO 2, and
∘ a heavy chain hypervariable region H3 that comprises the amino acid SEQ ID NO 11; and
• a VL domain, wherein said VL domain comprises:
∘ a heavy chain hypervariable region L1 that comprises the amino acid SEQ ID NO 5; and
∘ a heavy chain hypervariable region L2 that comprises the amino acid SEQ ID NO 13; and
∘ a heavy chain hypervariable region L3 that comprises the amino acid SEQ ID NO 14.

2. The chimeric antigen receptor (CAR) according to claim 1, comprising a VH domain comprising SEQ ID NO: 12, and a VL domain comprising SEQ ID NO: 15, or a variant thereof having at least an 85% sequence identity with SEQ ID NO 12 or 15, respectively.

3. The chimeric antigen receptor (CAR) according to claim 1, wherein the CD79b-targeting moiety is an antibody or scFv.

4. The chimeric antigen receptor (CAR) according to any of claim 1 to 3, that comprises:
• a transmembrane domain bound to a Hinge domain comprising SEQ ID NO: 27 or a variant thereof, wherein the variant thereof has at least a 95% sequence identity with SEQ ID NO 27;
• a costimulatory signaling domain comprising SEQ ID NO: 28 or a variant thereof, wherein the variant thereof has at least a 95% sequence identity with SEQ ID NO 28; and
• an intracellular signaling domain comprising SEQ ID NO: 29 or a variant thereof, wherein the variant thereof has at least a 95% sequence identity with SEQ ID NO 29.

5. A nucleic acid encoding the CAR according to any one of claims 1- 4.

6. A cell comprising the nucleic acid according to claim 5 and/or the CAR according to any one of claims 1-4.

7. The cell according to claim 6, wherein the cell is a T-cell.

8. A pharmaceutical composition comprising a plurality of cells according to claims 6 or 7 and a pharmaceutically acceptable carrier or diluent.

9. The cell according to claims 6 or 7 or the pharmaceutical composition according to claim 8 for use as a medicament.

10. The cells according to claims 6 or 7 or the pharmaceutical composition according to claim 8 for use in a method of treating Non-Hodgkin lymphoma wherein the method comprises administering the cell or composition to a patient in need thereof.

## Patentansprüche

1. Chimärer Antigenrezeptor (CAR) umfassend einen CD79b-Targeting-Anteil, welcher Folgendes umfasst:
• eine VH-Domäne, wobei die genannte VH-Domäne Folgendes umfasst:
∘ eine hypervariable Region der schweren Kette H1, welche die Aminosäure-SEQ ID NO 10 umfasst; und
∘ eine hypervariable Region der schweren Kette H2, welche die Aminosäure-SEQ ID NO 2 umfasst, und
∘ eine hypervariable Region der schweren Kette H3, welche die Aminosäure-SEQ ID NO 11 umfasst; und
• eine VL-Domäne, wobei die genannte VL-Domäne Folgendes umfasst:
∘ eine hypervariable Region der schweren Kette L1, welche die Aminosäure-SEQ ID NO 5 umfasst; und
∘ eine hypervariable Region der schweren Kette L2, welche die Aminosäure-SEQ ID NO 13 umfasst; und
∘ eine hypervariable Region der schweren Kette L3, welche die Aminosäure-SEQ ID NO 14 umfasst.

2. Chimärer Antigenrezeptor (CAR) nach Anspruch 1, umfassend eine VH-Domäne, welche SEQ ID NO: 12 umfasst, und eine VL-Domäne, welche SEQ ID NO: 15 umfasst, oder eine Variante derselben, welche mindestens 85 % Sequenzidentität jeweils mit SEQ ID NO: 12 oder 15 aufweist.

3. Chimärer Antigenrezeptor (CAR) nach Anspruch 1, wobei der CD79b-Targeting-Anteil ein Antikörper oder ScFv ist.

4. Chimärer Antigenrezeptor (CAR) nach einem der Ansprüche 1 bis 3, welcher Folgendes umfasst:
• eine Transmembrandomäne gebunden an einer Hinge-Domäne, welche SEQ ID NO: 27 oder eine Variante derselben umfasst, wobei die Variante derselben mindestens 95 % Sequenzidentität mit SEQ ID NO 27 aufweist;
• eine kostimulatorische Signaldomäne, welche SEQ ID NO: 28 oder eine Variante derselben umfasst, wobei die Variante derselben mindestens 95 % Sequenzidentität mit SEQ ID NO 28 aufweist; und
• eine intrazelluläre Signaldomäne, welche SEQ ID NO: 29 oder eine Variante derselben umfasst, wobei die Variante derselben mindestens 95 % Sequenzidentität mit SEQ ID NO 29 aufweist.

5. Nukleinsäure, welche den CAR nach einem der Ansprüche 1-4 kodiert.

6. Zelle, welche die Nukleinsäure nach Anspruch 5 und/oder den CAR nach einem der Ansprüche 1-4 umfasst.

7. Zelle nach Anspruch 6, wobei die Zelle eine T-Zelle ist.

8. Pharmazeutische Zusammensetzung, welche eine Vielzahl von Zellen nach den Ansprüchen 6 oder 7 und einen pharmazeutisch akzeptablen Trägerstoff oder ein pharmazeutisch akzeptables Verdünnungsmittel umfasst.

9. Zelle nach den Ansprüchen 6 oder 7 oder pharmazeutische Zusammensetzung nach Anspruch 8 für deren Verwendung als Medikament.

10. Zellen nach den Ansprüchen 6 oder 7 oder pharmazeutische Zusammensetzung nach Anspruch 8 für deren Verwendung in einem Verfahren zur Behandlung von Non-Hodgkin-Lymphom, wobei das Verfahren das Verabreichen der Zelle oder Zusammensetzung an einen Patienten, der es benötigt, umfasst.

## Revendications

1. Récepteur antigénique chimérique (CAR) comprenant une fraction de ciblage de CD79b, qui comprend :
• un domaine VH, dans lequel ledit domaine VH comprend :
∘ une région hypervariable de chaîne lourde H1 qui comprend l'acide aminé SEQ ID NO 10 ; et
∘ une région hypervariable de chaîne lourde H2 qui comprend l'acide aminé SEQ ID NO 2, et
∘ une région hypervariable de chaîne lourde H3 qui comprend l'acide aminé SEQ ID NO 11 ; et
• un domaine VL, dans lequel ledit domaine VL comprend :
∘ une région hypervariable de chaîne lourde L1 qui comprend l'acide aminé SEQ ID NO 5 ; et
∘ une région hypervariable de chaîne lourde L2 qui comprend l'acide aminé SEQ ID NO 13 ; et
∘ une région hypervariable de chaîne lourde L3 qui comprend l'acide aminé SEQ ID NO 14.

2. Récepteur antigénique chimérique (CAR) selon la revendication 1, comprenant un domaine VH comprenant SEQ ID NO : 12, et un domaine VL comprenant SEQ ID NO : 15, ou un variant de ceux-ci présentant au moins 85 % d'identité de séquence par rapport à SEQ ID NO 12 ou 15, respectivement.

3. Récepteur antigénique chimérique (CAR) selon la revendication 1, dans lequel la fraction de ciblage de CD79b est un anticorps ou scFv.

4. Récepteur antigénique chimérique (CAR) selon l'une quelconque des revendications 1 à 3, qui comprend :
• un domaine transmembranaire lié à un domaine charnière comprenant SEQ ID NO : 27 ou un variant de celui-ci, dans lequel le variant de celui-ci présente au moins 95 % d'identité de séquence par rapport à SEQ ID NO 27 ;
• un domaine de signalisation costimulateur comprenant SEQ ID NO : 28 ou un variant de celui-ci, dans lequel le variant de celui-ci présente au moins 95 % d'identité de séquence par rapport à SEQ ID NO 28 ; et
• un domaine de signalisation intracellulaire comprenant SEQ ID NO : 29 ou un variant de celui-ci, dans lequel le variant de celui-ci présente au moins 95 % d'identité de séquence par rapport à SEQ ID NO 29.

5. Acide nucléique codant le CAR selon l'une quelconque des revendications 1-4.

6. Cellule comprenant l'acide nucléique selon la revendication 5 et/ou le CAR selon l'une quelconque des revendications 1-4.

7. Cellule selon la revendication 6, dans laquelle la cellule est un lymphocyte T.

8. Composition pharmaceutique comprenant une pluralité de cellules selon les revendications 6 ou 7 et un véhicule ou diluant pharmaceutiquement acceptable.

9. Cellule selon les revendications 6 ou 7 ou composition pharmaceutique selon la revendication 8 pour son utilisation en tant que médicament.

10. Cellules selon les revendications 6 ou 7 ou composition pharmaceutique selon la revendication 8 pour leur utilisation dans une méthode de traitement de lymphome non hodgkinien, dans lesquelles la méthode comprend l'administration de la cellule ou la composition à un patient en ayant besoin.
